# EUROPEAN PATENT APPLICATION

(11) **EP 1 477 160 A2**
(43) Date of publication of application: **17.11.2004**
(21) Application number: 04011194.0
(22) Date of filing: 11.05.2004
(51) Int. Cl.: A61K 7/48, A61K 7/02

(54) **Makeup cosmetic and skin cosmetic**

(30) Priority: 13.05.2003 JP 2003134383
(71) Applicant: Seiwa Kasei Company, Limited, Higashi-Osaka, Osaka (JP)
(72) Inventor: Yoshioka, Masato c/o Seiwa Kasei Company,Limited, Higashiosaka-shi Osaka (JP); Takidani, Aiko c/o Seiwa Kasei Company, Limited, Higashiosaka-shi Osaka (JP); Adachi, Takashi c/o Seiwa Kasei Company, Limited, Higashiosaka-shi Osaka (JP)
(74) Representative: VOSSIUS & PARTNER

(57) **Abstract**

A makeup cosmetic and a skin cosmetic, which is produced by the following process and has a viscosity in a specific range,
wherein said process comprises:
polycondensing one or more kind selected from silylated peptides represented by the general formula (I) with one or more kind selected from silane compounds represented by the general formula (II) in an aqueous solution to form a polycondensed polymer, and then adding by addition reaction a silane compound represented by the general formula (III) in an aqueous solution to said polycondensed polymer;
wherein said silylated peptide represented by the following general formula (I) is 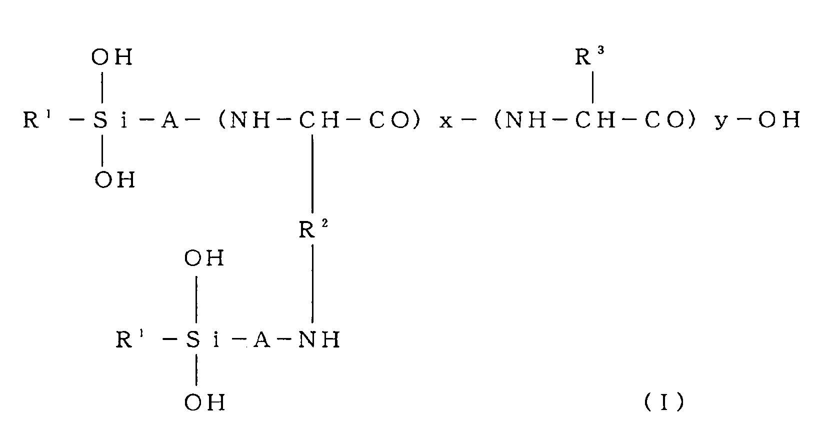 in which R¹ represents a hydroxy group etc., R² represents a residual group of a side chain obtained by removing the terminal end amino group of a basic amino acid having an amino group at the end of a side chain, R³ represents a side chain of an amino acid other than R², A is a connecting moiety, and x is from 0 to 50, y is from 1 to 100 and x+y is from 1 to 100;
wherein said silane compound represented by the following general formula (II) is

R⁴mSi(OH)pY(4-p-m) (II)

in which m represents an integer from 0 to 2, p represents an integer from 2 to 4, m+p is not more than 4, R⁴ represents an organic group; and
wherein said silane compound represented by the following general formula (III) is

R⁵ ₃Si(OH) (III)

wherein the three R⁵s represent organic groups.

## Description

The present invention relates to makeup cosmetics such as rouges, mascaras, eye liners and eye shadows, more particularly, to liquid and solid makeup cosmetics excellent in extensibility and affinity to the skin, lips and eyelashes, imparting smoothness, feeling of moistness, softness and moisture retention effect on the skin, lips and eyelashes, manifesting high close adherence, causing a tendency of no discoloration, and requiring little re-makeup.

The present invention also relates to skin cosmetics such as milky lotions and creams, more particularly, to skin cosmetics excellent in extensibility and affinity to skins, imparting smoothness, feeling of moistness, softness and unstickiness on the skin.

Makeup cosmetics are cosmetics intending change of aspects and healthy and beautiful appearance. Mascaras and eye liners dominantly include those in the form of liquid, and eye shadows are in the form of powder or cream and most of rouges are in the form of stick, pallet and liquid.

As functions required for makeup cosmetics, e.g. excellent coloration, excellent color retention, tendency of no discoloration are listed. Recently, also a feeling of moistness of moistness is required. However, when coloration is enhanced, viscosity increases to decrease extensibility, smooth application becomes impossible, and when a tendency of no discoloration is improved, coloration lowers and, scabrousness, dry feeling and roughness occur on skin, as generally known.

Therefore, there has been an attempt to solve these problems by compounding acrylic polymers or silicone resin (JP 2003-160434 A1). However, in the case of compounding of these acrylic polymers or silicone resin, there is a problem of stimulation by a remaining monomer, and compounding of water and water-soluble components is almost impossible, resultantly, it is difficult to obtain a feeling of moistness. Namely, simultaneous manifestation of both effects of moisture retention and tendency of no discoloration is not attained yet.

Regarding skin cosmetics, hydrolyzed peptides obtained by hydrolysis of natural proteins such as a collagen, keratin, silk protein, milk protein, soybean protein and wheat protein are compounded in the skin cosmetics, for imparting smoothness and feeling of moistness (JP 61-2046 B, JP 4-1118 A1, JP 8-198725 A1, JP 2002-241257 A1).

Although such hydrolyzed peptides are excellent in the effect of imparting feeling of moistness on the skin, they, however, tend to cause stickiness, when compounded in a large amount. As the result, they are hard to be compounded in an amount sufficient for imparting their effect.

High molecular silicones or silicone derivatives are also compounded in skin cosmetics for imparting smoothness and repellent property and improving extensibility on application (JP 5-262618 A1, JP 6-72853 A1) . However high molecular silicones do not impart feeling of moistness and cause oily feeling, when used in a large amount, although they impart smoothness on the skin.

There has been an attempt to solve the problem by compounding a hydrolyzed protein and a high molecular silicone in a skin cosmetic. However, since hydrolyzed proteins are hydrophilic and high molecular silicones are usually hydrophobic, on applying them on skin, a high molecular silicone hardly adheres to a portion of skin where a hydrolyzed protein is adhered, and a hydrolyzed protein is hardly adhered to a portion of skin where a silicone coating layer is formed. As the result, it is difficult to exhibit the effects of a hydrolyzed protein and a high molecular silicone, simultaneously.

An object of the present invention is to solve the problems in conventional makeup cosmetics as described above and to provide a makeup cosmetic having excellent affinity, extensibility and close adherence to the skin, lips and eyelashes, and capable of imparting a tendency of no discoloration, together with a smoothness, feeling of moistness, softness and moisture retention effect.

Another object of the present invention is to solve the problems in conventional skin cosmetics as described above and to provide a skin cosmetic having excellent affinity, extensibility and capable of imparting a smoothness, feeling of moistness, softness and unstickiness on the skin.

These objects could be achieved according to the present invention by the provision of a makeup cosmetic and a skin cosmetic comprising a specific silylated peptide-silane compound copolymer composition.

It has been found that a makeup cosmetic, such as rouges, mascaras, eye liners and eye shadows and a skin cosmetic, such as milky lotions and creams which are prepared by employing a specific silylated peptide-silane compound copolymer composition, have excellent extensibility and affinity to the skin, lips and eyelashes, and imparts smoothness, feeling of moistness, softness, unstickiness and a tendency of no discoloration.

The present invention provides a makeup cosmetic comprising a silylated peptide-silane compound copolymer composition which is produced by the following process and has a viscosity in the range from 500 to 30000 mPa · s in 70% of solid content concentration of said copolymer composition at 20 °C, wherein said process comprises:
polycondensing one or more kind selected from silylated peptides represented by the general formula (I) with one or more kind selected from silane compounds represented by the general formula (II) in an aqueous solution in a range of reaction molar ratio of said silylated peptide to said silane compound from 1:1 to 1:100 to form a polycondensed polymer, and then adding by addition reaction a silane compound represented by the general formula (III) in an aqueous solution to said polycondensed polymer;
wherein said silylated peptide represented by the following general formula (I) is 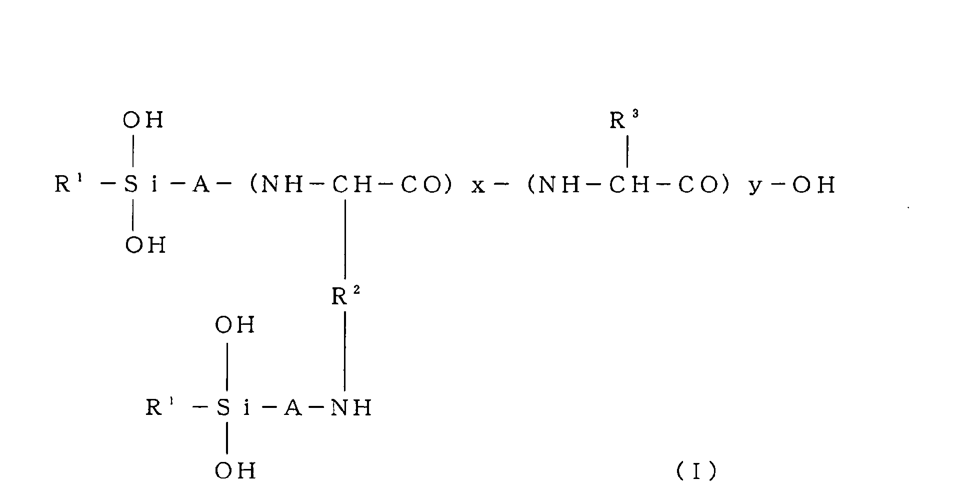 in which R¹ represents a hydroxy group or an alkyl group having 1 to 3 carbon atoms, R² represents a residual group of a side chain obtained by removing the terminal end amino group of a basic amino acid having an amino group at the end of a side chain, R³ represents a side chain of an amino acid other than R², A is a connecting moiety and represents at least one group selected from
-CH₂- , -(CH₂)₃- , -(CH₂)₃OCH₂CH(OH)CH₂-, -(CH₂)₃S-, -(CH₂)₃NH- and -(CH₂)₃OCOCH₂CH₂- , and x is from 0 to 50, y is from 1 to 100 and x+y is from 1 to 100, wherein x and y represent only the number of amino acid units and do not represent the order of amino acid sequence;
wherein said silane compound represented by the following general formula (II) is

R⁴mSi(OH)pY(4-p-m) (II)

in which m represents an integer from 0 to 2, p represents an integer from 2 to 4, m+p is not more than 4, R⁴ represents an organic group in which a carbon atom is directly connected to the silicon atom and R⁴s of m may be the same or different, and Ys of (4-p-m) represent an alkoxy group or hydrogen atom; and
wherein said silane compound represented by the following general formula (III) is

R⁵ ₃Si(OH) (III)

wherein the three R⁵s represent organic groups in which a carbon atom is directly connected to the silicon atom and the three R⁵s may be the same or different.

The letters of x and y used in the above described general formula (I) and of m, p and (4-p-m) used in the above described general formula (II) are the substitute characters.

The present invention also provides a skin cosmetic comprising a silylated peptide-silane compound copolymer composition which is produced by the following process and has a viscosity in the range from 500 to 20000 mPa · s in 70% of solid content concentration of said copolymer composition at 20 °C, wherein said process comprises:
polycondensing one or more kind selected from silylated peptides represented by the general formula (I) with one or more kind selected from silane compounds represented by the general formula (II) in an aqueous solution in a range of reaction molar ratio of said silylated peptide to said silane compound from 1:1 to 1:100 to form a polycondensed polymer, and then adding by addition reaction a silane compound represented by the general formula (III) in an aqueous solution to said polycondensed polymer.

The silylated peptide-silane compound copolymer composition used for the makeup cosmetics or skin cosmetics of the present invention may be produced by the methods, for example, disclosed in JP-A 2001-48732 and in JP-A 2001-48775. A method for producing said silylated peptide-silane compound copolymer composition is described in more detail hereinafter.

A silylated peptide represented by the above described general formula (I), which is one of components of the silylated peptide-silane compound copolymer composition, is easily produced in an aqueous solution by the methods disclosed in JP-A 8-59424 and JP-A 8-67608.

In the silylated peptide represented by the above described general formula (I), R² is a residual group of a side chain obtained by removing the terminal end amino group of a basic amino acid having an amino group at the end of a side chain. Examples of above described basic amino acid having an amino group at the end of a side chain include lysine, arginine and hydroxylysine.

R³ represents an amino acid side chain other than R². Examples of the amino acid include glutamic acid, aspartic acid, alanine, serine, threonine, valine, methionine, leucine, isoleucine, tyrosine, phenylalanine, proline and hydroxyproline.

In the silylated peptide shown by the above described formula (I), x is from 0 to 50, preferably from more than 0 to not more than 10 , y is from 1 to 100, preferably from 1 to 50, and x+y is from 1 to 100, preferably from 2 to 50. If x is more than the above range, because the number of the silyl group bonding to the amino acid in the side chains increases, the sorption action to the hair that the peptide originally possesses is decreased. If y is more than the above range, because the proportion of the silyl group portion to the peptide portion is small, the effects based on the silyl group portion cannot be sufficiently exerted. If the x+y is more than the above range, the sorbing ability and permeability to be exerted by the peptide is decreased in comparison with a low molecular weight peptide. The above described x, y and x+y are theoretically an integer. However, in case that the peptide portion is derived from a hydrolyzed peptide, since the hydrolyzed peptide is obtained in the form of a mixture of those having a different molecular weight, the measured values become average values.

The peptide portion of the silylated peptide represented by the above described formula (I) is illustrated with natural peptide, synthetic peptide, a hydrolyzed peptide obtained by partially hydrolyzing a protein with an acid, an alkali or an enzyme, or the mixture thereof. Among them, hydrolyzed peptide is preferably used according to the reason that the protein can be conveniently obtained and the number average molecular weight of the peptide portion can be easily controlled.

Examples of the hydrolyzed peptide include animal- or vegetable-derived protein such as collagen (including gelatin of modified product thereof), keratin, silk fibroin (silk), sericin, casein, conchiolin, elastin, yolk protein and albumen protein of eggs such as fowl and duck, soybean protein, wheat protein, corn protein, rice (rice bran) protein and potato protein; yeast proteins separated from yeasts such as yeasts belonging to genera Saccharomyces, Candida and Endomycopsis, or yeasts such as so-called beer yeasts and sake yeasts; and peptides obtained by partially hydrolyzing microorganism-derived proteins such as proteins separated from fungi (Basidiomycetes) or Chlorella with acid, alkali or enzyme, or the mixture thereof.

A silane compound, which is another of components of the silylated peptide-silane compound copolymer composition used for the makeup cosmetics or skin cosmetics of the present invention, is represented by the above described general formula (II), and said compound is obtained by hydrolyzing a silane compound represented by the following general formula (IV) in aqueous solution;

R⁶nSiX(4-n) (IV)

wherein n represents an integer from 0 to 2, R⁶ represents an organic group in which a carbon atom is directly connected to the silicon atom and R⁶s of n may be the same or different, and Xs of (4-n) represent at least a group selected from the group consisting of a hydroxyl group, an alkoxy group and a halogen group. The letters of n and (4-n) used in the above described general formula (IV) are the substitute characters.

Specific examples of such a silane compound represented by the general formula (IV) include tetramethoxysilane, methyltrimethoxysilane, methyldimethoxysilane, dimethyldimethoxysilane, phenyltrimethoxysilane, diphenyldimethoxysilane, hexyltrimethoxysilane, decyltrimethoxysilane, vinyltrimethoxysilane, 3-methacryloxypropyltrimethoxysilane, 3-methacryloxypropylmethyldimethoxysilane, N-(2-aminoethyl)-3-aminopropyltrimethoxysilane, N-(2-aminoethyl)-3-aminopropylmethyldimethoxysilane, 3-aminopropyltrimethoxysilane, N-phenyl-3-aminopropyltrimethoxysilane, 3-chloropropyltrimethoxysilane, 3-chloropropylmethyldimethoxysilane, 3-mercaptopropyltrimethoxysilane, 3-glycidoxypropyltrimethoxysilane, 3-glycidoxypropylmethyldimethoxysilane, dimethyloctadecyl[3-(trimethoxysilyl)propyl] ammonium chloride, 3-(trimethoxysilyl)propylpolyoxyethylene (10) ether, tetraethoxysilane, methyltriethoxysilane, methyldiethoxysilane, dimethyldiethoxysilane, phenyltriethoxysilane, diphenyldiethoxysilane, vinyltriethoxysilane, 3-methacryloxypropyltriethoxysilane, 3-methacryloxypropylmethyldiethoxysilane, N-(2-aminoethyl)-3-aminopropyltriethoxysilane, N-(2-aminoethyl)-3-aminopropylmethyldiethoxysilane, 3-aminopropyltriethoxysilane, 3-chloropropyltriethoxysilane, 3-chloropropylmethyldiethoxysilane, 3-glycidoxypropyltriethoxysilane, 3-glycidoxypropylmethyldiethoxysilane, 3-isocyanatepropyltriethoxysilane, methyldichlorosilane, methyltrichlorosilane, dimethyldichlorosilane, phenyltrichlorosilane, diphenyldichlorosilane, vinyltrichlorosilane and 3-chloropropylmethyldichlorosilane.

The reaction of the silylated peptides represented by the above described general formula (I) with the silane compound represented by the general formula (II) is carried out according to, for example, the following procedure:
Preparing an aqueous solution of the silylated peptides represented by the above described general formula (I) to become acidic with e.g. hydrochloric acid or sulfuric acid, or to become basic with e.g. an aqueous sodium hydroxide solution or an aqueous potassium hydroxide solution;
Adding dropwisely the silane compound represented by the general formula (IV) to said solution to form a silane compound represented by the general formula (II) having at least two hydroxyl groups directly connected to a silicon atom by hydrolyzing e.g. an alkoxy group or a halogen group of the silane compound represented by the above described general formula (IV);
Neutralizing the solution obtained thereby to polycondense a hydroxyl group of the organic silane compound having a hydrophilic group represented by the general formula (I) with a hydroxyl group of the silane compound represented by the general formula (II).

Thus, a silylated peptide-silane compound copolymer composition is obtained.

The hydrolysis reaction generally proceeds in a state of pH 0.5 to 3, preferably pH 1 to 3. However, because certain kinds of silylated peptide represented by the general formula (I) is likely to form insoluble materials in acidic state, when such silylated peptide is used, the hydrolysis is preferably conducted in the state of pH 10 to 11. When an alkoxysilane compound is used as the silane compound represented by the general formula (IV), pH control is required only before adding dropwisely the alkoxysilane compound. However, when the reaction is conducted in basic state by using a halogenated silane compound or carboxysilane compound as the silane compound represented by the general formula (IV), it is necessary to keep pH between 10 and 11 by adding e.g. an aqueous sodium hydroxide solution or an aqueous potassium hydroxide solution due to pH of the reaction solution decreasing during the reaction. Further, when the reaction is conducted in acidic state by using an amino silane compound as the silane compound represented by the general formula (IV), it is necessary to keep pH between 0.5 and 3, preferably 1 and 3 by adding diluted hydrochloric acid, diluted sulfuric acid or the like due to pH of the reaction solution increasing during the reaction.

The reaction temperature is preferably from 20 to 70 °C, more preferably from 30 to 60 °C , because the reaction does not proceed smoothly when the reaction temperature is too low, and because an alkoxy group or a halogen group of the silane compound represented by the above described general formula (IV) is hydrolyzed steeply when the reaction temperature is too high. In terms of the reaction time, though it varies depending on the amount of the reaction solution, it is preferable to add dropwisely the silane compound represented by the above described general formula (IV) to said solution for from 2 to 10 hours, more preferably from 3 to 6 hours, and then the solution is kept stirred for from 5 to 20 hours after said addition completed.

At completion of the hydrolysis reaction, because the reaction solution is in acidic or basic state, the solution is neutralized by adding an aqueous alkali solution such as an aqueous sodium hydroxide solution or aqueous potassium hydroxide solution when the reaction solution is acidic, or by adding an acid aqueous solution such as dilute hydrochloric acid or dilute sulfuric acid when the reaction solution is basic; and then the solution is stirred to be neutralized. By this neutralization, polycondensation is further proceeded to give a silylated peptide-silane compound copolymer composition. Stirring time conducted after the neutralization is preferably for from 1 to 10 hours.

The silylated peptide-silane compound copolymer composition used for a makeup cosmetic or a skin cosmetic of the present invention is produced by polycondensing one or more kind selected from silylated peptide represented by the above described general formula (I) with one or more kind selected from silane compound represented by the above described general formula (II) preferably in a range of reaction molar ratio of from 1:1 to 1:100, more preferably from 1:1 to 1:85.

When the reaction molar ratio of the silylated peptide represented by the general formula (I) to the silane compound represented by the general formula (II) is less than the above described range, a sufficient smoothness, a sufficient feeling of moistness and sufficient emulsification ability may not be obtained. When the reaction molar ratio of the silylated peptide represented by the general formula (I) to the silane compound represented by the general formula (II) is more than the above described range, silicon-oil touch may be caused and further handling ability may be deteriorated because of high viscosity of the silylated peptide-silane compound copolymer composition.

Because a hydroxyl group remains at terminal silylate group of the silylated peptide-silane compound copolymer composition obtained by above described procedure, the silylated peptide-silane compound copolymer compositions may coagulate themselves and, as a result, be highly polymerized. Therefore, a silane compound represented by the above described general formula (III) which produces with hydrolysis one hydroxyl group directly connecting to silicon atom is reacted with said copolymer composition.

The silane compound represented by the above described general formula (III) is obtained by hydrolyzing a silane compound represented by the following general formula (V) in aqueous solution

R⁷ ₃SiZ (V)

wherein three R⁷s represent organic groups in which a carbon atom is directly connected to the silicon atom and three R⁷s may be the same or different, Z is at least one group selected from a hydroxyl group, an alkoxy group and a halogen group.

Examples of the silane compound represented by the general formula (V) which produces with hydrolysis one hydroxyl group directly connecting to the silicon atom, include dimethylvinylchlorosilane, n-butyldimethylchlorosilane, tert-butyldimethylchlorosilane, tert-butyldiphenylchlorosilane, octadecyldimethylchlorosilane, methyldiphenylchlorosilane, tri-n-butylchlorosilane, triethylchlorosilane, trimethylchlorosilane, tri-n-propylchlorosilane, triphenylchlorosilane, trimethyliodidesilane, trimethylethoxysilane, dimethylvinylethoxysilane, dimethylvinylmethoxysilane, trimethylethoxysilane, trimethylmethoxysilane and triphenylethoxysilane.

In addition, silyl compounds having two silicon atoms such as hexamethyldisilazane can also be used because these compounds generate one hydroxyl group directly connecting to a silicon atom with hydrolysis.

Since a silane compound represented by the general formula (V) which produces by hydrolysis one hydroxyl group directly connecting to silicon atom has only one reactive group directly connected to a silicon atom; a silane compound represented by the general formula (III) obtained by hydrolyzing above said silane compound reacts with a hydroxyl group existing in the silylated peptide-silane compound copolymer composition; and reduces the hydroxyl group in said silylated peptide-silane compound copolymer composition; and thus prevents said silylated peptide-silane compound copolymer composition from further polycondensing by themselves. Namely, by reacting the silane compound represented by the general formula (III), a silylated peptide-silane compound copolymer composition having excellent storage stability can be obtained.

A reaction of a silylated peptide-silane compound copolymer composition with a silane compound represented by the general formula (III) is conducted, for example, by adding dropwisely the silane compound represented by the general formula (V) into an aqueous solution of the silylated peptide-silane compound copolymer composition; and thus a hydroxyl group of the silylated peptide-silane compound copolymer composition is condensed with a hydroxyl group of the silane compound represented by the general formula (III).

In case using a silane compound, in which Z represents a halogen atom, as the silane compound represented by the above described general formula (V) , because said silane compound shows excellent hydrolysis property, the above-described reaction can be conducted by directly adding dropwisely the silane compound represented by the above described general formula (V) into an aqueous solution of the silylated peptide-silane compound copolymer composition. However, in case using a silane compound, in which Z represents an alkoxy group or a silane compound containing two silicon atoms such as hexamethyldisilazane, as the silane compound represented by the above described general formula (V), it is necessary that said silane compound is previously hydrolyzed in an aqueous solution having pH 2 to 3 to give a silane compound represented by the general formula (III) and then the silane compound obtained by hydrolysis is added dropwisely into an aqueous solution of the silylated peptide-silane compound copolymer composition.

The reaction temperature of an silylated peptide-silane compound copolymer composition with a silane compound represented by the above described general formula (V) is preferably from 30 to 70 °C, more preferably from 30 to 60 °C. The reaction time, though it varies depending on the amount of reaction solution, is preferably for from 30 minutes to 2 hours to add dropwisely the silane compound represented by the general formula (V), and then for from 1 to 6 hours to stir the reaction solution after said addition completed.

After completion of the stirring, the reaction solution is neutralized with an aqueous alkali solution such as an aqueous sodium hydroxide solution or an aqueous potassium hydroxide solution; and the reaction is completed by further stirring for 1 to 10 hours to obtain a silylated peptide-silane compound copolymer composition. After controlling pH and concentration of the reaction solution, the silylated peptide-silane compound copolymer composition having viscosity in a range from 500 to 30000 mPa · s in 70% of solid content concentration of said copolymer composition at 20 °C is used for a makeup cosmetic of the present invention, and the silylated peptide-silane compound copolymer composition having viscosity in a range from 500 to 20000 mPa · s in 70% of solid content concentration of said copolymer composition at 20°C is used for a skin cosmetic of the present invention.

The reason why an amount of the silylated peptide-silane compound copolymer composition contained in a makeup cosmetic or a skin cosmetic of the present invention is set in the range in terms of the viscosity ranging from 500 to 30000 mPa · s for a makeup cosmetic or from 500 to 20000 mPa · s for a skin cosmetic in 70% of solid content concentration of said copolymer composition at 20 °C is;
that the effect of silylated peptide-silane compound copolymer composition imparting smoothness, feeling of moistness, softness, unstickiness on skins, lips and eyelashes and causing a tendency of no discoloration may not be realized due to insufficient polymerization when the viscosity in 70% of solid content concentration of said copolymer composition at 20 °C is less than 500 mPa · s ; and
that a handling ability of the silylated peptide-silane compound copolymer composition is deteriorated due to the lack of fluidity when the viscosity is more than the above described range, that is, 30000 mPa · s for a makeup cosmetic and 20000 mPa · s for a skin cosmetic.

The makeup cosmetics of the present invention include e.g. liquid or solid rouges, mascaras, eye liners and eye shadows,
and comprise a silylated peptide-silane compound copolymer composition obtainable by a process mentioned above.

Amount of the silylated peptide-silane compound copolymer composition contained in the makeup cosmetic of the present invention (that is, the blended amount in the makeup cosmetic) is preferably from 0.5 to 80% by weight, more preferably from 1 to 70% by weight. It may be unable to fully obtain the effects imparting feeling of moistness, softness and high close adherence to skins, eyelashes and lips, and causing a tendency of no discoloration, when the amount contained of the silylated peptide-silane compound copolymer composition is less than the above described range. Stickiness and deterioration of the touch may be caused when the amount contained of the silylated peptide-silane compound copolymer composition is more than the above described range. In blending to the makeup cosmetic, the silylated peptide-silane compound copolymer composition may be used alone or in a mixture of more than two kinds.

The makeup cosmetic of the present invention, which includes liquid and solid rouges, mascaras, eye liners and eye shadows may further comprise a volatile oil in addition to the silylated peptide-silane compound copolymer composition. When the volatile oil is used together, effects of close adherence and tendency of no discoloration are more remarkably manifested.

The volatile oil contained in the makeup cosmetic of the present invention is not particularly restricted, and preferable examples thereof include volatile isoparaffins, light isoparaffins, light liquid isoparaffins, liquid isoparaffins; decamethylcyclopentasiloxane, octamethylcyclotetrasiloxane and dodecamethylcyclohexasiloxane.

The content of the volatile oil in the makeup cosmetic of the present invention (compounding amount into makeup cosmetic) is preferably from 1 to 70 wt%, more preferably from 5 to 50 wt% in the makeup cosmetic. The reason for this is that when the content of this volatile oil is over the above-mentioned range, there is a possibility that an effect of imparting smoothness, feeling of moistness and softness on the skin, eyelashes and lips cannot be expected, and when below the above-mentioned range, there is a possibility that an effect of imparting a tendency of no discoloration together with close adherence may not be fully manifested. The volatile oil may be used singly or in admixture of two or more.

The makeup cosmetic of the present invention may further comprise other suitable components in addition to the silylated peptide-silane compound copolymer composition and, if any, the volatile oil in the range not to deteriorate the effects of the present invention.

Example of the other suitable component include anionic surfactants, nonionic surfactants, cationic surfactants, ampholytic surfactants, cationic polymers, ampholytic polymers, anionic polymers, thickener, waxes, hydrocarbons, ester oils, glycerides, extracts from animals and vegetables, polysaccharide and derivatives thereof, hydrolyzed peptides of proteins derived from animals, vegetables and microorganism or derivatives thereof, wetting agent, lower alcohols, higher alcohols, amino acids, non-volatile fats and oils, non-volatile silicones, silicas, inorganic powders, dyes, pigments, preservatives and perfumes.

The skin cosmetics of the present invention include milky lotions, hand creams and massage creams and comprise a silylated peptide-silane compound copolymer composition obtainable by a process mentioned above.

Amount of the silylated peptide-silane compound copolymer composition contained in the skin cosmetic of the present invention (that is, the blended amount in the skin cosmetic) is preferably from 0.05 to 10% by weight, more preferably from 0.1 to 5% by weight.

When the amount contained of the silylated peptide-silane compound copolymer composition is more than the above described range, it may cause stickiness on the skin due to the water-insoluble property of the silylated peptide-silane compound copolymer composition and to accumulation of said copolymer composition in daily use. On the other hand, it may be unable to fully obtain the effects imparting smoothness, feeling of moistness, softness and unsticky feeling on the skin, when the amount contained of the silylated peptide-silane compound copolymer composition is less than the above described range. In blending to the skin cosmetic, the silylated peptide-silane compound copolymer composition may be used alone or in a mixture of more than two kinds.

The skin cosmetic of the present invention may further comprise other suitable components in addition to the silylated peptide-silane compound copolymer composition in the range not to deteriorate the effects of the present invention.

Example of the other suitable component include anionic surfactants, nonionic surfactants, cationic surfactants, ampholytic surfactants, cationic polymers, ampholytic polymers, anionic polymers, thickener, extracts from animals and vegetables, polysaccharide and derivatives thereof, hydrolyzed peptides of proteins derived from animals, vegetables and microorganism or derivatives thereof, wetting agent, lower alcohols, higher alcohols, amino acids, fats and oils, silicones, preservatives and perfumes.

The makeup cosmetic of the present invention is excellent in extensibility and affinity to theskin, lips and eyelashes, and manifests excellent effects imparting smoothness, feeling of moistness and softness, and causing a tendency of no discoloration.

The skin cosmetic of the present invention is excellent in extensibility and affinity to theskin, and manifests excellent effects imparting smoothness, feeling of moistness, softness and unstickiness on the skin.

The present invention is more specifically described and explained by means of the following Examples. The examples should not be construed to limit the scope to the present invention. In the following Examples and Comparative Examples, the amount of each component blended is represented by the part by weight; and if the amount blended is not the amount of solid content of said component, the concentration of said solid content is represented by being enclosed in parentheses followed to said component name. The percent (%) showing concentration is % by weight. Prior to the Examples and Comparative Examples, examples producing a silylated peptide-silane compound copolymer composition used in the Examples and Comparative Examples are described as Production Example.

### Production Example 1

### Production of N-[2-hydroxy-3-(3'-methyldihydroxysilyl)propoxy]propyl hydrolyzed sericin-dimethyldiethoxysilane-octyltriethoxysilane copolymer composition [1:25:25 (molar ratio)]

Into the reaction vessel made of glass shaped in cylinder with round bottom of its inner diameter of 12 cm and its volume of 2 liters, 200 g of 10% aqueous solution of N-[2-hydroxy-3-(3'-methyldihydroxysilyl)propoxy]propyl hydrolyzed sericin (wherein the number average molecular weight of said hydrolyzed sericin is about 500) and 11.5 g of 18% aqueous solution of hydrochloric acid were added to adjust pH of a mixture to 1.5 and said mixture was heated up to 60 °C. While the mixture being stirred in 400 rpm, the mixture of 99.1 g of dimethyldiethoxysilane (a trade name of KBE-22, manufactured by Shin-Etsu Silicone Co. , Ltd. ) and 184.7 g of octyltriethoxysilane (a trade name of A-137, Nippon Unicar Co., Ltd. ) was dropped into said mixture for 5 hours, and then, after said dropping being completed, the solution obtained was further stirred for 15 hours at 60 °C. Then, while the solution being stirred, 40.8 g of 5% aqueous solution of sodium hydroxide was gradually dropped into said solution to adjust the solution pH to 6, and the solution was further stirred for one hour at 60 °C. While the reaction solution obtained being stirred in 400 rpm at 60 °C, 11.6 g of trimethylchlorosilane (a trade name of KA-31, manufactured by Shin-Etsu Silicone Co., Ltd.) was added, and then said reaction solution was stirred for one hour at 60 °C. Then, after 78.8 g of 5% aqueous solution of sodium hydroxide being dropped into the reaction solution and pH of said solution being adjusted to 6, the solution was stirred for one hour at 60 °C, and then the solution was heated up to 80 °C and further stirred for one hour. Then, the reaction solution was concentrated to the solid content of 70% by vacuum concentration with the rotary-evaporator.
Thus, 260 g of N-[2-hydroxy-3-(3'-methyldihydroxysilyl)propoxy]propyl hydrolyzed sericin-dimethyldiethoxysilane-octyltriethoxysilane copolymer composition was obtained.

The viscosity of 70% aqueous solution at 20 °C of obtained N-[2-hydroxy-3-(3'-methyldihydroxysilyl)propoxy]propyl hydrolyzed sericin-dimethyldiethoxysilane-octyltriethoxysilane copolymer composition was measured by B-type viscometer with the rotor 3 and number of revolution 30, thus the viscosity resulted in 2120 mPa · s.

### Production Example 2

### Production of N-[2-hydroxy-3-(3'-methyldihydroxysilyl)propoxy]propyl hydrolyzed collagen-dimethyldiethoxysilane-octyltriethoxysilane copolymer composition [1:25:25 (molar ratio)]

Into the reaction vessel made of glass shaped in cylinder with round bottom of its volume of 2 liters, 150 g of 10% aqueous solution of
N-[2-hydroxy-3-(3'-methyldihydroxysilyl)propoxy]propyl hydrolyzed collagen (wherein the number average molecular weight of hydrolyzed collagen is about 500) and 7.6 g of 18% aqueous solution of hydrochloric acid were added to adjust pH of the mixture to 1.5 and said mixture was heated up to 60 °C . While the mixture being stirred in 400 rpm, the mixture of 79.7 g of dimethyldiethoxysilane (a trade name of KBE-22, manufactured by Shin-Etsu Silicone Co. , Ltd. ) and 148.6 g of octyltriethoxysilane (a trade name of A-137, Nippon Unicar Co., Ltd.) was dropped into said mixture for 5 hours, and then, after said dropping being completed, the solution obtained was further stirred for 15 hours at 60 °C. Then, while the solution being stirred, 22.9 g of 5% aqueous solution of sodium hydroxide was gradually dropped into said solution to adjust the solution pH to 6, and the solution was further stirred for one hour at 60 °C. While the reaction solution obtained being stirred in 400 rpm at 60 °C, 9.3 g of trimethylchlorosilane (a trade name of KA-31, manufactured by Shin-Etsu Silicon Co., Ltd.) was added, and then said reaction solution was stirred for one hour at 60 °C. Then, after 68.5 g of 5% aqueous solution of sodium hydroxide being dropped into the reaction solution and pH of said solution being adjusted to 6, the solution was stirred for one hour at 60 °C, and then the solution was heated up to 80 °C and further stirred for one hour. Then, the reaction solution was concentrated to the solid content of 70% by vacuum concentration with the rotary-evaporator.
Thus, 211.9 g of
N-[2-hydroxy-3-(3'-methyldihydroxysilyl)propoxy]propyl hydrolyzed collagen-dimethyldiethoxysilane-octyltriethoxysilane copolymer composition was obtained.

The viscosity of 70% aqueous solution at 20 °C of obtained N-[2-hydroxy-3-(3'-methyldihydroxysilyl)propoxy]propyl hydrolyzed collagen-dimethyldiethoxysilane-octyltriethoxysilane copolymer composition was measured according to the same condition applied in Production Example 1, thus the viscosity resulted in 1,116 mPa · s.

### Production Example 3

### Production of N-[2-hydroxy-3-(3'-methyldihydroxysilyl)propoxy]propyl hydrolyzed silk-dimethyldiethoxysilane-octyltriethoxysilane copolymer composition [1:40:40 (molar ratio)]

Into the reaction vessel made of glass shaped in cylinder with round bottom of its volume of 2 liters, 127.3 g of 10% aqueous solution of
N-[2-hydroxy-3-(3'-methyldihydroxysilyl)propoxy]propyl hydrolyzed silk (wherein the number average molecular weight of hydrolyzed silk is about 600) and 4.8 g of 18% aqueous solution of hydrochloric acid were added to adjust pH of the mixture to 1.5 and said mixture was heated up to 60 °C. While the mixture being stirred in 400 rpm, the mixture of 88.0 g of dimethyldiethoxysilane (a trade name of KBE-22, manufactured by Shin-Etsu Silicon Co., Ltd.) and 164.0 g of octyltriethoxysilane (a trade name of A-137, Nippon Unicar Co. , Ltd.) was dropped into said mixture for 5 and half hours, and then, after said dropping being completed, the solution obtained was further stirred for 15 hours at 60 °C. Then, while the solution being stirred, 17.1 g of 5% aqueous solution of sodium hydroxide was gradually dropped into said solution to adjust the solution pH to 6, and the solution was further stirred for one hour at 60 °C. While the reaction solution obtained being stirred in 400 rpm at 60 °C, 6.4 g of trimethylchlorosilane (a trade name of KA-31, manufactured by Shin-Etsu Silicon Co., Ltd.) was added, and then said reaction solution was stirred for one hour at 60 °C. Then, after 45.7 g of 5% aqueous solution of sodium hydroxide being dropped into the reaction solution and pH of said solution being adjusted to 6, the solution was stirred for one hour at 60 °C, and then the solution was heated up to 80 °C and further stirred for one hour. Then, the reaction solution was concentrated to the solid content of 70% by vacuum concentration with the rotary-evaporator.
Thus, 209 g of
N-[2-hydroxy-3-(3'-methyldihydroxysilyl)propoxy]propyl hydrolyzed silk-dimethyldiethoxysilane-octyltriethoxysilane copolymer composition was obtained.

The viscosity of 70% aqueous solution at 20 °C of obtained N-[2-hydroxy-3-(3'-methyldihydroxysilyl)propoxy]propyl hydrolyzed silk-dimethyldiethoxysilane-octyltriethoxysilane copolymer composition was measured by the same condition applied in Production Example 1, thus the viscosity resulted in 640 mPa · s.

### Production Example 4

### Production of N-[2-hydroxy-3-(3'-methyldihydroxysilyl)propoxy]propyl hydrolyzed silk-dimethyldiethoxysilane-octyltriethoxysilane copolymer composition [1:25:25 (molar ratio)]

Into the reaction vessel made of glass shaped in cylinder with round bottom of its volume of 2 liters, 407.5 g of 5% aqueous solution of
N-[2-hydroxy-3-(3'-methyldihydroxysilyl)propoxy]propyl hydrolyzed silk (wherein the number average molecular weight of hydrolyzed silk is about 600) and 13.0 g of 18% aqueous solution of hydrochloric acid were added to adjust pH of the mixture to 1.2 and said mixture was heated up to 60 °C. While the mixture being stirred in 400 rpm, the mixture of 88.0 g of dimethyldiethoxysilane (a trade name of KBE-22, manufactured by Shin-Etsu Silicon Co., Ltd.) and 164.0 g of octyltriethoxysilane (a trade name of A-137, Nippon Unicar Co., Ltd. ) was dropped into said mixture for 5 and half hours, and then, after said dropping being completed, the solution obtained was further stirred for 15 hours at 60 °C. Then, while the solution being stirred, 36.7 g of 5% aqueous solution of sodium hydroxide was gradually dropped into said solution to adjust the solution pH to 6, and the solution was further stirred for one hour at 60 °C. While the reaction solution obtained being stirred in 400 rpm at 60 °C, 10.3 g of trimethylchlorosilane (a trade name of KA-31, manufactured by Shin-Etsu Silicon Co., Ltd.) was added, and then said reaction solution was stirred for one hour at 60 °C. Then, after 72.4 g of 5% aqueous solution of sodium hydroxide being dropped into the reaction solution and pH of said solution being adjusted to 6, the solution was stirred for one hour at 60 °C, and then the solution was heated up to 80 °C and further stirred for one hour. Then, the reaction solution was concentrated to the solid content of 70% by vacuum concentration with the rotary-evaporator.
Thus, 220 g of
N-[2-hydroxy-3-(3'-methyldihydroxysilyl)propoxy]propyl hydrolyzed silk-dimethyldiethoxysilane-octyltriethoxysilane copolymer composition was obtained.

The viscosity of 70% aqueous solution at 20 °C of obtained N-[2-hydroxy-3-(3'-methyldihydroxysilyl)propoxy]propyl hydrolyzed silk-dimethyldiethoxysilane-octyltriethoxysilane copolymer composition was measured by the same condition applied in Production Example 1, thus the viscosity resulted in 8,050 mPa · s.

### Production Example 5

### Production of N-[2-hydroxy-3-(3'-methyldihydroxysilyl)propoxy]propyl hydrolyzed soybean protein-dimethyldiethoxysilane-octyltriethoxysilane copolymer composition [1:25:25 (molar ratio)]

Into the reaction vessel made of glass shaped in cylinder with round bottom of its volume of 2 liters, 196 g of 10% aqueous solution of
N-[2-hydroxy-3-(3'-methyldihydroxysilyl)propoxy]propyl hydrolyzed soybean protein ( wherein the number average molecular weight of hydrolyzed soybean protein is about 600) and 17.7 g of 18% aqueous solution of hydrochloric acid were added to adjust pH of the mixture to 1.5 and said mixture was heated up to 60 °C. While the mixture being stirred in 400 rpm, the mixture of 86.2 g of dimethyldiethoxysilane (a trade name of KBE-22, manufactured by Shin-Etsu Silicon Co., Ltd.) and 160.7 g of octyltriethoxysilane (a trade name of A-137, Nippon Unicar Co., Ltd.) was dropped into said mixture for 8 hours, and then, after said dropping being completed, the solution obtained was further stirred for 15 hours at 60 °C. Then, while the solution being stirred, 54.3 g of 5% aqueous solution of sodium hydroxide was gradually dropped into said solution to adjust the solution pH to 6, and the solution was further stirred for one hour at 60 °C. While the reaction solution obtained being stirred in 400 rpm at 60 °C, 10.1 g of trimethylchlorosilane (a trade name of KA-31, manufactured by Shin-Etsu Silicon Co., Ltd.) was added, and then said reaction solution was stirred for one hour at 60 °C. Then, after 73.7 g of 5% aqueous solution of sodium hydroxide being dropped into the reaction solution and pH of said solution being adjusted to 6, the solution was stirred for one hour at 60 °C, and then the solution was heated up to 80 °C and further stirred for one hour. Then, the reaction solution was concentrated to the solid content of 70% by vacuum concentration with the rotary-evaporator.
Thus, 222.7 g of
N-[2-hydroxy-3-(3'-methyldihydroxysilyl)propoxy]propyl hydrolyzed soybean protein-dimethyldiethoxysilane-octyltriethoxysilane copolymer composition was obtained.

The viscosity of 70% aqueous solution at 20°C of obtained N-[2-hydroxy-3-(3'-methyldihydroxysilyl)propoxy]propyl hydrolyzed soybean protein-dimethyldiethoxysilane-octyltriethoxysilane copolymer composition was measured by the same condition applied in Production Example 1, thus the viscosity resulted in 3,560 mPa • s.

### Production Example 6

### Production of N-[2-hydroxy-3-(3'-methyldihydroxysilyl)propoxy]propyl hydrolyzed wheat protein-dimethyldiethoxysilane-octyltriethoxysilane copolymer composition [1:15:15 (molar ratio)]

Into the reaction vessel made of glass shaped in cylinder with round bottom of its volume of 2 liters, 345.3 g of 10% aqueous solution of
N-[2-hydroxy-3-(3'-methyldihydroxysilyl)propoxy]propyl hydrolyzed wheat protein (wherein the number average molecular weight of hydrolyzed soybean protein is about 600) and 26.8 g of 18% aqueous solution of hydrochloric acid were added to adjust pH of the mixture to 1.5 and said mixture was heated up to 60 °C. While the mixture being stirred in 400 rpm, the mixture of 81 g of dimethyldiethoxysilane (a trade name of KBE-22, manufactured by Shin-Etsu Silicon Co. , Ltd. ) and 151 g of octyltriethoxysilane (a trade name of A-137, Nippon Unicar Co., Ltd.) was dropped into said mixture for 5 hours, and then, after said dropping being completed, the solution obtained was further stirred for 15 hours at 60 °C. Then, while the solution being stirred, 73.2 g of 5% aqueous solution of sodium hydroxide was gradually dropped into said solution to adjust the solution pH to 6, and the solution was further stirred for one hour at 60 °C. While the reaction solution obtained being stirred in 400 rpm at 60 °C, 11.9 g of trimethylchlorosilane (a trade name of KA-31, manufactured by Shin-Etsu Silicon Co., Ltd.) was added, and then said reaction solution was stirred for one hour at 60 °C. Then, after 87.4 g of 5% aqueous solution of sodium hydroxide being dropped into the reaction solution and pH of said solution being adjusted to 6, the solution was stirred for one hour at 60 °C, and then the solution was heated up to 80 °C and further stirred for one hour. Then, the reaction solution was concentrated to the solid content of 70% by vacuum concentration with the rotary-evaporator.
Thus, 233 g of
N-[2-hydroxy-3-(3'-methyldihydroxysilyl)propoxy]propyl hydrolyzed wheat protein-dimethyldiethoxysilane-octyltriethoxysilane copolymer composition was obtained.

The viscosity of 70% aqueous solution at 20 °C of obtained N-[2-hydroxy-3-(3'-methyldihydroxysilyl)propoxy]propyl hydrolyzed wheat protein-dimethyldiethoxysilane-octyltriethoxysilane copolymer composition was measured by the same condition applied in Production Example 1, thus the viscosity resulted in 14,340 mPa · s.

### Example 1 and Comparative Example 1

Two kinds of solid rouges of compositions shown in Table 1 were prepared and their moisturizing effect was evaluated. In Example 1, an N-[2-hydroxy-3-(3'-methyldihydroxysilyl)propoxy]propyl hydrolyzed sericin-dimethyldiethoxysilane-octyltriethoxysilane copolymer composition obtained in Production Example 1 was used. In Comparative Example 1, the silylated peptide-silane compound copolymer composition was not used.

**Table 1**

| | Example 1 | Comparative Example 1 |
|---|---|---|
| Silylated peptide-silane compound copolymer composition obtained in Production Example 1 (70%) | 15.0 | 0.0 |
| | | |
| Candelilla wax | 5.0 | 5.0 |
| | | |
| Micro crystalline wax *1 | 3.0 | 3.0 |
| | | |
| Sericin *2 | 5.0 | 5.0 |
| | | |
| Vaseline *3 | 3.0 | 3.0 |
| | | |
| Dimethylpolysiloxane 10 cs | 10.0 | 10.0 |
| | | |
| Dimethylpolysiloxane 10,000 cs | 0.0 | 5.0 |
| | | |
| Neopentyl glycol dicaprate | 35.95 | 35.95 |
| | | |
| Polyglyceryl-2 triisostearate | 20.0 | 20.0 |
| | | |
| Red No. 201 | 1.0 | 1.0 |
| | | |
| Red No. 202 | 2.0 | 2.0 |
| | | |
| Propyl p-hydroxybenzoate | 0.05 | 0.05 |

| | | |
|---|---|---|
| *1: Multiwax W-445 (trade name) manufactured by Witco | | |
| *2: Ceresin #76 (trade name) manufactured by La Ceresin | | |
| *3: Sun White P-200 (trade name) manufactured by Nikko Rika K.K. | | |

The moisturizing effect in application using the above-mentioned rouges of Example 1 and Comparative Example 1 was measured by the following method.

### [Test method of moisturizing effect]

The amount of water in the horny layer of the inside part of a forearm of a subject kept under rest condition in a room of a temperature of 23°C and a humidity of 47% for 30 minutes was measured as skin conductance (*µ*S) by SKICON-200 EX (trade name) manufactured by IBS (K.K.), and used as a value before the test. Next, the rouges of Example 1 and Comparative Example 1 were applied on separate portions of 3 cm square each in an amount of 0.2 mg, and after 3 minutes, the amount of water in the horny layer was measured and this value was used as a value at initiation of the test (0 minute). Subsequently, the amount of water in the horny layer was measured with the lapse of time until 130 minutes. The number of the measuring points was 5 every each time, and the average value was used as the amount of water in the horny layer.

The change by lapse of time of the skin conductance (*µ* S) of skin on which the above-mentioned rouges of Example 1 and Comparative Example 1 were applied is shown in Table 2.

**Table 2**

| Time (min) | Example 1 | Comparative example 1 |
|---|---|---|
| Before the test | 46.0 | 48.0 |
| 0 | 80.4 | 50.0 |
| 4 | 65.2 | 48.0 |
| 9 | 60.4 | 48.0 |
| 12 | 60.6 | 48.0 |
| 16 | 62.2 | 49.0 |
| 20 | 59.8 | 46.4 |
| 30 | 58.2 | 44.0 |
| 40 | 59.8 | 48.0 |
| 50 | 61.2 | 49.4 |
| 60 | 60.8 | 50.8 |
| 90 | 56.2 | 49.8 |
| 130 | 52.0 | 47.6 |

As shown in Table 2, skin on which the rouge of Example 1 containing the N-[2-hydroxy-3-(3'-methyldihydroxysilyl)propoxy]propyl hydrolyzed sericin-dimethyldiethoxysilane-octyltriethoxysilane copolymer composition obtained in Production Example 1 had been applied had high value of skin conductance at initiation of the test (0 minute), and after 9 minutes, showed lower value and thereafter, approximately constant value. However, its value was kept at higher level as compared with that before the test (46.0). On the other hand, skin on which the rouge of Comparative Example 1 had been applied had a value of skin conductance at initiation of the test (0 minute) somewhat higher as compared with that before the test, however, after 4 minutes, its value decreased to approximately the same value as before the test, and thereafter, the value scarcely varied. From this results, it was evident that the N-[2-hydroxy-3-(3'-methyldihydroxysilyl)propoxy]propyl hydrolyzed sericin-dimethyldiethoxysilane-octyltriethoxysilane copolymer composition contained in the rouge of Example 1 enhanced the moisturizing effect of a horny layer of skin.

### Examples 2 to 3 and Comparative Example 2

Three kinds of liquid rouges of compositions shown in Table 3 were prepared, and easiness of extension (extensibility), conformability (affinity) and smoothness in application, and close adherence, feeling of moistness of lip, softness and tendency of no discoloration after application, were evaluated.

Example 2 uses the N-[2-hydroxy-3-(3'-methyldihydroxysilyl)propoxy]propyl hydrolyzed silk-dimethyldiethoxysilane-octyltriethoxysilane copolymer composition obtained in Production Example 4 as a silylated peptide-silane compound copolymer composition and light isoparaffin which is a volatile oil. Example 3 uses only the silylated peptide-silane compound copolymer composition obtained in Production Example 4, and Comparative Example 2 uses a poly(oxyethylene·oxypropylene)methylpolysiloxane copolymer which is a polyether-modified silicone instead of the silylated peptide-silane compound copolymer composition.

**Table 3**

| | Example 2 | Example 3 | Comparative Example 2 |
|---|---|---|---|
| Silylated peptide-silane compound copolymer composition obtained in Production Example 4 (70%) | 80.0 | 96.95 | 0.0 |
| | | | |
| Light isoparaffin *4 | 11.95 | 0.0 | 0.0 |
| | | | |
| Polyether-modified silicone *5 | 0.0 | 0.0 | 91.95 |
| | | | |
| Red No. 201 | 1.0 | 1.0 | 1.0 |
| | | | |
| Red No. 202 | 2.0 | 2.0 | 2.0 |
| | | | |
| Propyl p-hydroxybenzoate | 0.05 | 0.05 | 0.05 |

| | | | |
|---|---|---|---|
| *4: Pearl Learn 4 (trade name) manufactured by NOF Corp. | | | |
| *5: SH3775M (trade name) manufactured by Toray Dowcorning Silicone. | | | |

Regarding the above-mentioned rouges of Examples 2 to 3 and Comparative Example 2, easiness of extension, conformability and smoothness in applying on lips and close adherence of rouge, feeling of moistness of lips, softness and tendency of no discoloration after application were evaluated under the following evaluation standard by 10 panelists. The results are shown as the average value of 10 panelists in Table 4.

### Evaluation standard

| | |
|---|---|
| Strongly felt | 3 point |
| Felt | 2 point |
| Scarcely felt | 1 point |
| Not felt utterly | 0 point |

**Table 4**

| | Example 2 | Example 3 | Comparative example 2 |
|---|---|---|---|
| (in applying on lips) Easiness of extension | 2.5 | 2.3 | 1.0 |
| Conformability | 3.0 | 2.5 | 1.5 |
| Smoothness | 2.5 | 2.3 | 2.0 |
| (after application) Close adherence | 2.8 | 2.4 | 2.0 |
| Feeling of moistness | 2.5 | 2.8 | 1.2 |
| Softness | 2.6 | 2.3 | 1.5 |
| Tendency of no discoloration | 2.7 | 1.8 | 1.0 |

As shown in Table 4, the rouge of Example 3 showed higher evaluation values in all of the evaluation items of easiness of extension, conformability and smoothness in application and close adherence, feeling of moistness of lip, softness and tendency of no discoloration after application, as compared with the rouge of Comparative Example 2 in which a poly(oxyethylene·oxypropylene)methylpolysiloxane copolymer which is a polyether-modified silicone had been compounded instead of the silylated peptide-silane compound copolymer composition.

In Example 2 in which the silylated peptide-silane compound copolymer composition and light isoparaffin which is a volatile oil were used together, although evaluation of feeling of moistness was poor as compared with Example 3, higher evaluation results were obtained in the other evaluation items, particularly in tendency of no discoloration after application.

The tendency of no discoloration in Examples 2 to 3 and Comparative Example 2 was evaluated also by the following method. Namely, the above-mentioned rouges of Examples 2 to 3 and Comparative Example 2 were applied each in an amount of 0.5 mg on an artificial leather [artificial leather SACLARE (trade name) manufactured by Idemitsu Technofine] of 4 cm square, and left for 5 minutes. Thereafter, the artificial leather on which the rouge had been applied was immersed in 10 ml of olive oil for 5 minutes, the artificial leather was removed, then, the degree of coloration of the olive oil was measured by the absorbance at a wavelength of 640 nm using a spectrophotometer.

The ratios of absorbance in Examples 2 to 3 when the absorbance in Comparative Example 2 was 100 are shown in Table 5, meaning that when the ratio of absorbance is larger, color is more dark, and a coloring matter in the rouge is dissolved in the olive oil. Namely, it means that when the value of absorbance is smaller, discoloration does not occur easily.

**Table 5**

| | Example 2 | Example 3 | Comparative example 2 |
|---|---|---|---|
| Ratio of absorbance (% ) | 52 | 72 | 100 |

As shown in Table 5, with the rouge in Example 2 using a silylated peptide-silane compound copolymer composition and light isoparaffin as a volatile oil, a higher evaluation result of tendency of no discoloration was obtained, as compared with the rouge of Example 3 using only silylated peptide-silane compound copolymer composition and in which a volatile oil had not been compounded, not to mention of the rouge of Comparative Example 2 in which a poly(oxyethylene·oxypropylene)methylpolysiloxane copolymer which is a polyether-modified silicone had been compounded instead of the silylated peptide-silane compound. Also, with the rouge of Example 3 using only the silylated peptide-silane compound copolymer composition and in which a volatile oil had not been compounded, the ratio of absorbance was 72% as compared with the rouge of Comparative Example 2 in which the poly(oxyethylene·oxypropylene)methylpolysiloxane copolymer had been compounded, clarifying that it had a larger tendency of no discoloration as compared with rouges in which a conventional silicone had been compounded.

From these results, it was evident that the N-[2-hydroxy-3-(3'-methyldihydroxysilyl)propoxy]propyl hydrolyzed silk-dimethyldiethoxysilane-octyltriethoxysilane copolymer composition had excellent conformability (affinity) and close adherence to lips, and excellent in imparting a feeling of moistness and softness to lips, and that when a volatile oil is used together, tendency of no discoloration is further reinforced.

### Example 4 and Comparative Example 3

Two kinds of mascaras of compositions shown in Table 6 were prepared, and easiness of extension (extensibility), conformability (affinity) and smoothness of cream in application, and close adherence after application were evaluated.

Example 4 uses the N-[2-hydroxy-3-(3'-methyldihydroxysilyl)propoxy]propyl hydrolyzed collagen-dimethyldiethoxysilane-octyltriethoxysilane copolymer composition obtained in Production Example 2 as a silylated peptide-silane compound copolymer composition. Comparative Example 4 uses a poly(oxyethylene·oxypropylene)methylpolysiloxane copolymer which is a polyether-modified silicone instead of the silylated peptide-silane compound copolymer composition.

**Table 6**

| | Example 4 | Comparative example 3 |
|---|---|---|
| Silylated peptide-silane compound copolymer composition obtained in Production Example 2 (70%) | 10.0 | 0.0 |
| | | |
| Polyether-modified silicone *6 | 0.0 | 10.0 |
| | | |
| Micro crystalline wax *7 | 15.0 | 15.0 |
| | | |
| Light isoparaffin *8 | 32.95 | 32.95 |
| | | |
| Iron oxide | 12.0 | 12.0 |
| | | |
| Alkylacrylate copolymer emulsion *9 | 15.0 | 15.0 |
| | | |
| Sterilized ion-exchange water | 15.0 | 15.0 |
| | | |
| Propyl p-hydroxybenzoate | 0.05 | 0.05 |

| | | |
|---|---|---|
| *6: SH3775M (trade name) manufactured by Toray Dowcorning Silicone. | | |
| *7: Multiwax W-445 (trade name) manufactured by Witco | | |
| *8: Pearl Learn 4 (trade name) manufactured by NOF Corp. | | |
| *9: Jurymer ET410 (trade name) manufactured by NIHON JUNYAKU Co., Ltd. | | |

Regarding the above-mentioned mascaras of Example 4 and Comparative Example 3, easiness of extension, conformability and smoothness in applying on eyelashes and close adherence after application were evaluated under the same evaluation standard as that in Example 2 by 10 panelists. The results are shown as the average value of 10 panelists in Table 7.

**Table 7**

| | Example 4 | Comparative example 3 |
|---|---|---|
| (in applying on eyelashes) Easiness of extension | 2.6 | 2.0 |
| Conformability | 2.6 | 1.0 |
| Smoothness of cream | 2.4 | 1.6 |
| (after application) Close adherence | 2.8 | 2.0 |

As shown in Table 7, the mascara of Example 4 containing a silylated peptide-silane compound copolymer composition and light isoparaffin as a volatile oil showed higher evaluation values in all of the evaluation items, as compared with the mascara of Comparative Example 3 which contains a poly(oxyethylene·oxypropylene)methylpolysiloxane copolymer which is a polyether-modified silicone had been compounded instead of the silylated peptide-silane compound copolymer composition and the same volatile oil.

From these results, it was evident that the silylated peptide-silane compound copolymer composition is excellent in easiness of extension, conformability and smoothness of cream in applying on eyelashes, and close adherence after application.

### Example 5 and Comparative Example 4

Two kinds of eye liners of compositions shown in Table 8 were prepared, and easiness of extension (extensibility), conformability (affinity) and smoothness in application, and close adherence after application were evaluated.

Example 5 uses the N-[2-hydroxy-3-(3'-methyldihydroxysilyl)propoxy]propyl hydrolyzed sericin-dimethyldiethoxysilane-octyltriethoxysilane copolymer composition obtained in Production Example 1 as a silylated peptide-silane compound copolymer composition. Comparative Example 4 uses a poly(oxyethylene·oxypropylene)methylpolysiloxane copolymer which is a polyether-modified silicone instead of the silylated peptide-silane compound copolymer composition.

**Table 8**

| | Example 5 | Comparative example 4 |
|---|---|---|
| Silylated peptide-silane compound copolymer composition obtained in Production Example 1 (70%) | 5.0 | 0.0 |
| | | |
| Polyether-modified silicone *10 | 0.0 | 5.0 |
| | | |
| Iron oxide | 15.0 | 15.0 |
| | | |
| Vinyl acetate resin emulsion *11 | 40.0 | 40.0 |
| | | |
| Dipropylene glycol | 5.0 | 5.0 |
| | | |
| Sodium carboxymethyl cellulose | 2.0 | 2.0 |
| | | |
| Acetyl tributylcitrate | 1.0 | 1.0 |
| | | |
| Propyl p-hydroxybenzoate | 0.05 | 0.05 |
| | | |
| Sterilized ion-exchange water | 31.95 | 31.95 |

| | | |
|---|---|---|
| *10: SH3775M (trade name) manufactured by Toray Dowcorning Silicone. | | |
| *11: Polysol (trade name) manufactured by Showa Highpolymer Co. , Ltd. | | |

Regarding the above-mentioned eye liners of Example 5 and Comparative Example 4, easiness of extension, conformability and smoothness in applying and close adherence after application were evaluated under the same evaluation standard as that in Example 2 by 10 panelists. The results are shown as the average value of 10 panelists in Table 9.

**Table 9**

| | Example 5 | Comparative example 4 |
|---|---|---|
| (in applying on eyelashes) Easiness of extension | 2.4 | 2.1 |
| Conformability | 2.7 | 1.6 |
| Smoothness | 2.2 | 1.6 |
| (after application) Close adherence | 2.7 | 2.1 |

As shown in Table 9, the eye liner of Example 5 containing a silylated peptide-silane compound copolymer composition showed higher evaluation values in all of the evaluation items, as compared with the eye liner of Comparative Example 4 which contains a poly(oxyethylene·oxypropylene)methylpolysiloxane copolymer which is a polyether-modified silicone had been compounded instead of the silylated peptide-silane compound copolymer composition.

From these results, it was evident that the silylated peptide-silane compound copolymer composition is excellent in easiness of application, conformability and smoothness in applying on skin, and close adherence after application.

### Example 6 and Comparative Example 5

Two kinds of eye shadows of compositions shown in Table 10 were prepared, and easiness of extension (extensibility), conformability (affinity) and smoothness in application, and close adherence after application were evaluated.

Example 6 uses the N-[2-hydroxy-3-(3'-methyldihydroxysilyl)propoxy]propyl hydrolyzed silk-dimethyldiethoxysilane-octyltriethoxysilane copolymer composition obtained in Production Example 4 as a silylated peptide-silane compound copolymer composition. Comparative Example 5 uses a poly(oxyethylene·oxypropylene)methylpolysiloxane copolymer which is a polyether-modified silicone instead of the silylated peptide-silane compound copolymer composition.

**Table 10**

| | Example 6 | Comparative example 5 |
|---|---|---|
| Silylated peptide-silane compound copolymer composition obtained in Production Example 4 (70%) | 3.0 | 0.0 |
| | | |
| Polyether-modified silicone *12 | 0.0 | 3.0 |
| | | |
| Squalane | 7.0 | 7.0 |
| | | |
| Methylpolysiloxane 100cs | 3.0 | 3.0 |
| | | |
| Vitamin E | 0.05 | 0.05 |
| | | |
| Talc | 46.9 | 46.9 |
| | | |
| Mica | 12.0 | 12.0 |
| | | |
| Sericite | 3.0 | 3.0 |
| | | |
| Pigment | 16.0 | 16.0 |
| | | |
| Pearlescent agent | 12.0 | 12.0 |
| | | |
| Propyl p-hydroxybenzoate | 0.05 | 0.05 |

| | | |
|---|---|---|
| *12: SH3775M (trade name) manufactured by Toray Dowcorning Silicone. | | |

Regarding the above-mentioned eye shadows of Example 6 and Comparative Example 5, easiness of extension, conformability and smoothness in applying and close adherence after application were evaluated under the same evaluation standard as that in Example 2 by 10 panelists. The results are shown as the average value of 10 panelists in Table 11.

**Table 11**

| | Example 6 | Comparative example 5 |
|---|---|---|
| (in applying on eyelashes) Easiness of extension | 2.8 | 2.2 |
| Conformability | 2.9 | 1.8 |
| Smoothness | 2.2 | 1.8 |
| (after application) Close adherence | 2.8 | 2.2 |

As shown in Table 11, the eye shadow of Example 6 containing a silylated peptide-silane compound copolymer composition showed higher evaluation values in all of the evaluation items, as compared with the eye shadow of Comparative Example 5 which contains a poly(oxyethylene·oxypropylene)methylpolysiloxane copolymer which is a polyether-modified silicone had been compounded instead of the silylated peptide-silane compound copolymer composition.

From these results, it was evident that the silylated peptide-silane compound copolymer composition is excellent in easiness of application, conformability and smoothness in applying on skin, and close adherence after application.

### Example 7 and Comparative Example 6

Two kinds of mascaras of compositions shown in Table 12 were prepared, and easiness of extension (extensibility), conformability (affinity) and smoothness of cream in application, and close adherence after application were evaluated.

Example 7 uses the N-[2-hydroxy-3-(3'-methyldihydroxysilyl)propoxy]propyl hydrolyzed soybean protein-dimethyldiethoxysilane-octyltriethoxysilane copolymer composition obtained in Production Example 5 as a silylated peptide-silane compound copolymer composition. Comparative Example 6 uses a poly(oxyethylene·oxypropylene)methylpolysiloxane copolymer which is a polyether-modified silicone instead of the silylated peptide-silane compound copolymer composition.

**Table 12**

| | Example 7 | Comparative example 6 |
|---|---|---|
| Silylated peptide-silane compound copolymer composition obtained in Production Example 5 (70%) | 10.0 | 0.0 |
| | | |
| Polyether-modified silicone *13 | 0.0 | 10.0 |
| | | |
| Micro crystalline wax *14 | 15.0 | 15.0 |
| | | |
| Light isoparaffin *15 | 32.95 | 32.95 |
| | | |
| Iron oxide | 12.0 | 12.0 |
| | | |
| Alkylacrylate copolymer emulsion *16 | 15.0 | 15.0 |
| | | |
| Sterilized ion-exchange water | 15.0 | 15.0 |
| | | |
| Propyl p-hydroxybenzoate | 0.05 | 0.05 |

| | | |
|---|---|---|
| *13: SH3775M (trade name) manufactured by Toray Dowcorning Silicone. | | |
| *14: Multiwax W-445 (trade name) manufactured by Witco | | |
| *15: Pearl Learn 4 (trade name) manufactured by NOF Corp. | | |
| *16: Jurymer ET410 (trade name) manufactured by NIHON JUNYAKU Co . , Ltd. | | |

Regarding the above-mentioned mascaras of Example 7 and Comparative Example 6, easiness of extension, conformability and smoothness of cream in applying and close adherence after application were evaluated under the same evaluation standard as that in Example 2 by 10 panelists. The results are shown as the average value of 10 panelists in Table 13.

**Table 13**

| | Example 7 | Comparative example 6 |
|---|---|---|
| (in applying on eyelashes) Easiness of extension | 2.4 | 2.0 |
| Conformability | 2.7 | 1.2 |
| Smoothness of cream | 2.2 | 1.4 |
| (after application) Close adherence | 2.4 | 1.8 |

As shown in Table 13, the masacara of Example 7 containing a silylated peptide-silane compound copolymer composition showed higher evaluation values in all of the evaluation items, as compared with the mascara of Comparative Example 6 which contains a poly(oxyethylene·oxypropylene)methylpolysiloxane copolymer which is a polyether-modified silicone had been compounded.

From these results, it was evident that the silylated peptide-silane compound copolymer composition is excellent in easiness of application on eye lashes, conformability and smoothness in applying, and close adherence after application.

### Example 8 and Comparative Example 7

Two kinds of eye liners of compositions shown in Table 14 were prepared, and easiness of extension (extensibility), conformability (affinity) and smoothness in application, and close adherence after application were evaluated.

Example 8 uses the N-[2-hydroxy-3-(3'-methyldihydroxysilyl)propoxy]propyl hydrolyzed wheat protein-dimethyldiethoxysilane-octyltriethoxysilane copolymer composition obtained in Production Example 6 as a silylated peptide-silane compound copolymer composition. Comparative Example 7 uses a poly(oxyethylene-oxypropylene)methylpolysiloxane copolymer which is a polyether-modified silicone instead of the silylated peptide-silane compound copolymer composition.

**Table 14**

| | Example 8 | Comparative example 7 |
|---|---|---|
| Silylated peptide-silane compound copolymer composition obtained in Production Example 6 (70%) | 5.0 | 0.0 |
| | | |
| Polyether-modified silicone *17 | 0.0 | 5.0 |
| | | |
| Iron oxide | 15.0 | 15.0 |
| | | |
| Vinyl acetate resin emulsion *18 | 40.0 | 40.0 |
| | | |
| Dipropylene glycol | 5.0 | 5.0 |
| | | |
| Sodium carboxymethyl cellulose | 2.0 | 2.0 |
| | | |
| Acetyl tributylcitrate | 1.0 | 1.0 |
| | | |
| Propyl p-hydroxybenzoate | 0.05 | 0.05 |
| | | |
| Sterilized ion-exchange water | 31.95 | 31.95 |

| | | |
|---|---|---|
| *17: SH3775M (trade name) manufactured by Toray Dowcorning Silicone. | | |
| *18: Polysol (trade name) manufactured by Showa Highpolymer Co., Ltd. | | |

Regarding the above-mentioned eye liners of Example 8 and Comparative Example 7, easiness of extension, conformability an smoothness in applying and close adherence after application wer evaluated under the same evaluation standard as that in Exampl 2 by 10 panelists. The results are shown as the average valu of 10 panelists in Table 15.

**Table 15**

| | Example 8 | Comparative example 7 |
|---|---|---|
| (in applying on eyelashes) Easiness of extension | 2.3 | 2.0 |
| Conformability | 2.7 | 1.5 |
| Smoothness | 2.5 | 1.6 |
| (after application) Close adherence | 2.4 | 1.9 |

As shown in Table 15, the eye liner of Example 8 containing the silylated peptide-silane compound copolymer composition showed higher evaluation values in all of the evaluation items, as compared with the eye liner of Comparative Example 7 which contains a poly(oxyethylene-oxypropylene)methylpolysiloxane copolymer which is a polyether-modified silicone had been compounded.

From these results, it was evident that the silylated peptide-silane compound copolymer composition is excellent in easiness of application, conformability and smoothness in applying on skin, and close adherence after application.

### Example 9 and Comparative Example 8

Two kinds of hand creams of compositions shown in Table 16 were prepared and their moisturizing effect was evaluated. In Example 9, an N-[2-hydroxy-3-(3'-methyldihydroxysilyl)propoxy]propyl hydrolyzed sericin-dimethyldiethoxysilane-octyltriethoxysilane copolymer composition obtained in Production Example 1 was used. In Comparative Example 8, the silylated peptide-silane compound copolymer composition was not used.

**Table 16**

| | Example 9 | Comparative Example 8 |
|---|---|---|
| Silylated peptide-silane compound copolymer composition obtained in Production Example 1 (70%) | 3.0 | 0.0 |
| | | |
| Sodium acrylate/acryloyldimethyl Taurine copolymer +isohexadecane+ Polysorbate80 mixture *19 | 3.0 | 3.0 |
| | | |
| Squalane | 3.0 | 3.0 |
| | | |
| Sterilized ion-exchange water | Volume adjustable totally 100 part | Volume adjustable totally 100 part |

| | | |
|---|---|---|
| *19: Simulgel EG (trade name), manufactured by Seppic Co.,Ltd. | | |

The moisturizing effect in application using the above-mentioned hand cream of Example 9 and Comparative Example 8 was measured according to the same method for measuring moisturizing effect as that in Example 1, except that 0.2 ml of hand cream is used instead of 0.2 g of rouge.

The change by lapse of time of the skin conductance (*µ* S) of skin on which the above-mentioned hand creams of Example 9 and Comparative Example 8 were applied is shown in Table 17.

**Table 17**

| Time (min) | Example 9 | Comparative example 8 |
|---|---|---|
| Before the test | 47.0 | 50.0 |
| 0 | 380.0 | 105.4 |
| 4 | 125.0 | 58.2 |
| 9 | 74.0 | 54.4 |
| 12 | 81.0 | 56.6 |
| 16 | 78.0 | 54.2 |
| 20 | 79.4 | 58.8 |
| 30 | 67.0 | 49.2 |
| 40 | 68.0 | 51.8 |
| 50 | 72.4 | 52.2 |
| 60 | 72.8 | 55.8 |
| 90 | 64.8 | 49.2 |
| 130 | 65.6 | 51.0 |

As shown in Table 17, skin on which the hand cream of Example 9 containing the N-[2-hydroxy-3-(3'-methyldihydroxysilyl)propoxy]propyl hydrolyzed sericin-dimethyldiethoxysilane-octyltriethoxysilane copolymer composition obtained in Production Example 1 had been applied had high value of skin conductance at initiation of the test (0 minute), and after 9 minutes, showed lower value and thereafter, approximately constant value. However, its value was kept at higher level as compared with that before the test (47.0). On the other hand, skin on which the hand cream of Comparative Example 8 had been applied had a value of skin conductance at initiation of the test (0 minute) higher as compared with that before the test, however, after 4 minutes, its value decreased to approximately the same value as before the test, and thereafter, the value scarcely varied. From this results, it was evident that the N-[2-hydroxy-3-(3'-methyldihydroxysilyl)propoxy]propyl hydrolyzed sericin-dimethyldiethoxysilane-octyltriethoxysilane copolymer composition contained in the hand cream of Example 9 enhanced the moisturizing effect of a horny layer of skin.

### Example 10 and Comparative Examples 9 to 10

Three kinds of massage creams of compositions shown in Table 18 were prepared, and easiness of extension in application (extensibility), conformability (affinity) to skin and smoothness of cream, and feeling of moistness of skin, softness and unstickyness, were evaluated.

Example 10 uses the N-[2-hydroxy-3-(3'-methyldihydroxysilyl)propoxy]propyl hydrolyzed collagen-dimethyldiethoxysilane-octyltriethoxysilane copolymer composition obtained in Production Example 2 as a silylated peptide-silane compound copolymer composition. Comparative Example 9 uses a poly(oxyethylene·oxypropylene)methylpolysiloxane copolymer which is a polyether-modified silicone instead of the silylated peptide-silane compound copolymer composition. In Comparative Example 10, neither a silylated peptide-silane compound copolymer composition nor a silicone is used.

**Table 18**

| | Example 10 | Comparative Example | |
|---|---|---|---|
| | | 9 | 10 |
| Silylated peptide-silane compound copolymer composition obtained in Production Example 2 (70%) | 1.0 | 0.0 | 0.0 |
| | | | |
| Polyether-modified silicone *20 | 0 | 0.7 | 0 |
| | | | |
| Arachidyl glucoside+Arachidyl alcohol+Behenyl alcohol mixture *21 | 1.0 | 1.0 | 1.0 |
| | | | |
| Cetearyl glucoside+Cetearyl alcohol mixture *22 | 1.0 | 1.0 | 1.0 |
| | | | |
| Isopropyl isostearate | 3.0 | 3.0 | 3.0 |
| | | | |
| Hydrolyzed collagen liquid *23 | 0.1 | 0.1 | 0.1 |
| | | | |
| p-Hydroxybenzoic acid esters+Phenoxy ethanol +Diethyleneglycol ethylether mixture *24 | 0.3 | 0.3 | 0.3 |
| | | | |
| Sterilized ion-exchange water | Volume adjustable totally 100 part | Volume adjustable totally 100 part | Volume adjustable totally 100 part |

| | | | |
|---|---|---|---|
| *20: SH3775M (trade name) manufactured by Toray Dowcorning Silicone. | | | |
| *21: Montanov 202(trade name), manufactured by Seppic Co.,Ltd. | | | |
| *22: Montanov 68(trade name), manufactured by Seppic Co.,Ltd. | | | |
| *23: Promois WU-32R (trade name), manufactured by SEIWA KASEI Co. , Ltd. | | | |
| *24: Seisept H (trade name), manufactured by SEIWA KASEI Co . , Ltd. | | | |

Regarding the above-mentioned massage creams of Example 10 and Comparative Example 9 to 10, easiness of extension in applying on hand, conformability to skin and smoothness of cream, and feeling of moistness of skin, softness and unstickiness after application were evaluated under the following evaluation standard by 10 panelists. The results are shown as the average value of 10 panelists in Table 19.

### Evaluation standard

| | |
|---|---|
| Strongly felt | 3 point |
| Felt | 2 point |
| Scarcely felt | 1 point |
| Not felt utterly | 0 point |

**Table 19**

| | Example 10 | Comparative Example | |
|---|---|---|---|
| | | 9 | 10 |
| (in applying on hands) Easiness of extension | 2.5 | 2.0 | 2.0 |
| Conformability to skin | 3.0 | 1.5 | 1.0 |
| Smoothness of cream | 2.5 | 2.0 | 0.4 |
| (after application) Feeling of moistness | 2.5 | 1.2 | 1.0 |
| Softness | 2.0 | 1.5 | 0.7 |
| Unstickiness | 2.7 | 2.4 | 1.0 |

As shown in Table 19, the massage cream of Example 10 showed higher evaluation values in all of the evaluation items of easiness of extension in application, conformability to the skin, smoothness of cream and feeling of moistness of skin after application, softness and unstickiness, as compared with the massage cream of Comparative Example 9 in which a poly (oxyethylene · oxypropylene) methylpolysiloxane copolymer had been compounded and the massage cream of Comparative Example 10 in which neither silylated peptide-silane compounds nor silicones are compounded.

From these results, it was evident that the silylated peptide-silane compound copolymer composition is excellent in conformability to skin and effects imparting feeling of moistness of skin, smoothness and unstickiness as compared to a silicone, poly(oxyethylene·oxypropylene)methylpolysiloxane copolymer.

### Example 11 and Comparative Example 11

Two kinds of milky lotions of compositions shown in Table 20 were prepared, and easiness of extension in application and conformability to skin, and feeling of moistness of skin and unstickyness, were evaluated.

Example 11 uses the N-[2-hydroxy-3-(3'-methyldihydroxysilyl)propoxy]propyl hydrolyzed silk-dimethyldiethoxysilane-octyltriethoxysilane copolymer composition obtained in Production Example 3 as a silylated peptide-silane compound copolymer composition. Comparative Example 11 uses dimethyl polysiloxane instead of the silylated peptide-silane compound copolymer composition.

**Table 20**

| | Example 11 | Comparative Example 11 |
|---|---|---|
| Silylated peptide-silane compound copolymer composition obtained in Production Example 3 (70%) | 5.0 | 0.0 |
| | | |
| Dimethyl polysiloxane *25 | 0.0 | 3.5 |
| | | |
| Cyclomethicone *26 | 5.0 | 5.0 |
| | | |
| Liquid paraffin | 2.5 | 2.5 |
| | | |
| Squalane | 2.5 | 2.5 |
| | | |
| Dipropylene glycol | 1.0 | 1.0 |
| | | |
| Glycerin | 2.0 | 2.0 |
| | | |
| Carboxyvinyl polymer *27 | 0.1 | 0.1 |
| | | |
| Potassium hydroxide | 0.02 | 0.02 |
| | | |
| p-Hydroxybenzoic acid esters+Phenoxy ethanol +Diethyleneglycol ethylether mixture *28 | 0.3 | 0.3 |
| | | |
| Sterilized ion-exchange water | Volume adjustable totally 100 part | Volume adjustable totally 100 part |

| | | |
|---|---|---|
| *25: SH200C 10cs (trade name) manufactured by Toray Dowcorning Silicone. | | |
| *26: SH245 (trade name) manufactured by Toray Dowcorning Silicone. | | |
| *27: CARBOPOL980 (trade name), manufactured by Goodrich. | | |
| *28: Seisept H (trade name), manufactured by SEIWA KASEI Co. , Ltd. | | |

Regarding the above-mentioned milky lotions of Example 11 and Comparative Example 11, easiness of extension in applying the milky lotion on skin, conformability to skin and smoothness of cream, and feeling of moistness of skin, softness and unstickiness, after application, were evaluated under the same evaluation standard as that in Example 10. The results are shown as the average value of 10 panelists in Table 21.

**Table 21**

| | Example 10 | Comparative Example 9 |
|---|---|---|
| (in applying on skin) Easiness of extension | 2.6 | 2.0 |
| Conformability to skin | 2.6 | 1.0 |
| Smoothness of cream | 2.4 | 1.6 |
| (after application) Feeling of moistness | 2.8 | 1.0 |
| Softness | 2.4 | 1.6 |
| Unstickiness | 2.6 | 2.0 |

As shown in Table 21, the milky lotion of Example 11, in which the silylated peptide-silane compound copolymer composition had been compounded, showed higher evaluation values in all of the evaluation items, as compared with the milky lotion of Comparative Example 11 in which dimethylpolysiloxane had been compounded.

From these results, it was evident that the silylated peptide-silane compound copolymer composition is excellent in extensibility on skin (easiness of extension in application) and affinity (conformability to skin) and effects imparting feeling of moistness of skin, smoothness and unstickiness.

## Claims

1. A makeup cosmetic comprising a silylated peptide-silane compound copolymer composition which is produced by the following process and has a viscosity in the range from 500 to 30000 mPa · s in 70% of solid content concentration of said copolymer composition at 20 °C,
wherein said process comprises:
polycondensing one or more kind selected from silylated peptides represented by the general formula (I) with one or more kind selected from silane compounds represented by the general formula (II) in an aqueous solution in a range of reaction molar ratio of said silylated peptide to said silane compound from 1:1 to 1:100 to form a polycondensed polymer, and then adding by addition reaction a silane compound represented by the general formula (III) in an aqueous solution to said polycondensed polymer;
wherein said silylated peptide represented by the following general formula (I) is 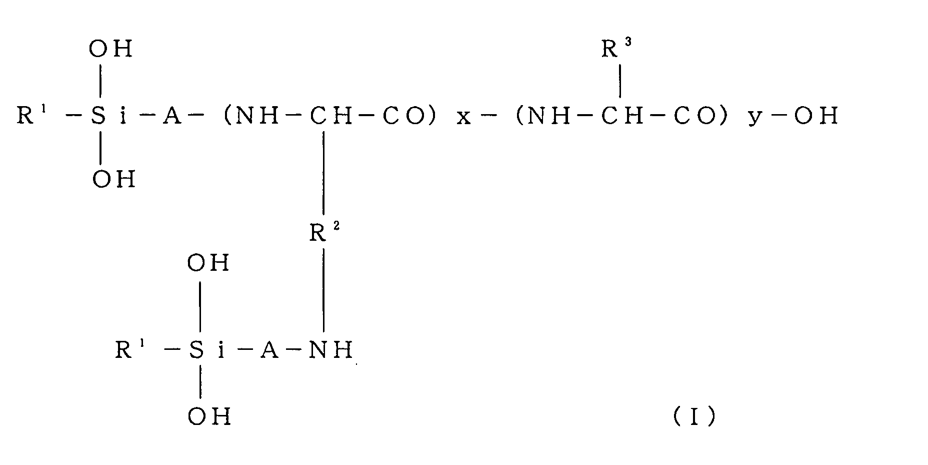 in which R¹ represents a hydroxy group or an alkyl group having 1 to 3 carbon atoms, R² represents a residual group of a side chain obtained by removing the terminal end amino group of a basic amino acid having an amino group at the end of a side chain, R³ represents a side chain of an amino acid other than R², A is a connecting moiety and represents at least one group selected from
-CH₂-, -(CH₂)₃-, -(CH₂)₃OCH₂CH(OH)CH₂-, -(CH₂)₃S-, -(CH₂)₃NH- and -(CH₂)₃OCOCH₂CH₂-, and x is from 0 to 50, y is from 1 to 100 and x+y is from 1 to 100, wherein x and y represent only the number of amino acid units and do not represent the order of amino acid sequence;
wherein said silane compound represented by the following general formula (II) is
R⁴mSi(OH)pY(4-p-m) (II)
in which m represents an integer from 0 to 2, p represents an integer from 2 to 4, m+p is not more than 4, R⁴ represents an organic group in which a carbon atom is directly connected to the silicon atom and R⁴s of m may be the same or different, and Ys of (4-p-m) represent an alkoxy group or hydrogen atom; and
wherein said silane compound represented by the following general formula (III) is
R⁵ ₃Si(OH) (III)
wherein the three R⁵s represent organic groups in which a carbon atom is directly connected to the silicon atom and the three R⁵s may be the same or different.

2. The makeup cosmetic according to Claim 1, wherein the content of the silylated peptide-silane compound copolymer composition is from 0.5 to 80% by weight in the makeup cosmetic.

3. The makeup cosmetic according to Claim 1 or Claim 2, which further comprises a volatile oil.

4. The makeup cosmetic according to Claim 3, wherein the content of the volatile oil is from 1 to 70 wt% in the makeup cosmetic.

5. The makeup cosmetic according to Claim 1, which is a rouge.

6. The makeup cosmetic according to Claim 1, which is a mascara.

7. The makeup cosmetic according to Claim 1, which is an eye liner.

8. The makeup cosmetic according to Claim 1, which is an eye shadow.

9. A skin cosmetic comprising a silylated peptide-silane compound copolymer composition which is produced by the following process and has aviscosityinthe range from 500 to 20000 mPa · s in 70% of solid content concentration of said copolymer composition at 20 °C,
wherein said process comprises:
polycondensing one or more kind selected from silylated peptides represented by the general formula (I) with one or more kind selected from silane compounds represented by the general formula (II) in an aqueous solution in a range of reaction molar ratio of said silylated peptide to said silane compound from 1:1 to 1:100 to form a polycondensed polymer, and then
adding by addition reaction a silane compound represented by the general formula (III) in an aqueous solution to said polycondensed polymer;
wherein said silylated peptide represented by the following general formula (I) is 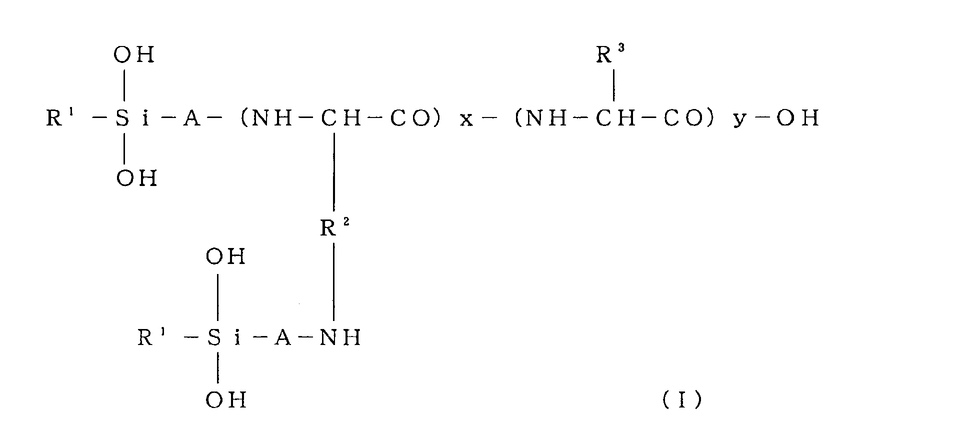 in which R¹ represents a hydroxy group or an alkyl group having 1 to 3 carbon atoms, R² represents a residual group of a side chain obtained by removing the terminal end amino group of a basic amino acid having an amino group at the end of a side chain, R³ represents a side chain of an amino acid other than R², A is a connecting moiety and represents at least one group selected from
-CH₂- , -(CH₂)₃- , -(CH₂)₃OCH₂CH(OH)CH₂-, (CH₂)₃S-, -(CH₂)₃NH- and -(CH₂)₃OCOCH₂CH₂-, and x is from 0 to 50, y is from 1 to 100 and x+y is from 1 to 100, wherein x and y represent only the number of amino acid units and do not represent the order of amino acid sequence;
wherein said silane compound represented by the following general formula (II) is
R⁴mSi(OH)pY(4-p-m) (II)
in which m represents an integer from 0 to 2, p represents an integer from 2 to 4, m+p is not more than 4, R⁴ represents an organic group in which a carbon atom is directly connected to the silicon atom and R⁴s of m may be the same or different, and Ys of (4-p-m) represent an alkoxy group or hydrogen atom; and
wherein said silane compound represented by the following general formula (III) is
R⁵ ₃Si(OH) (III)
wherein the three R⁵s represent organic groups in which a carbon atom is directly connected to the silicon atom and the three R⁵s may be the same or different.

10. The skin cosmetic according to Claim 9, wherein the content of the silylated peptide-silane compound copolymer composition is from 0.05 to 10% by weight in the skin cosmetic.

11. The skin cosmetic according to Claim 9, which is a milky lotion.

12. The skin cosmetic according to Claim 9, which is a cream.
